# EUROPEAN PATENT APPLICATION

(11) **EP 2 594 639 A1**
(43) Date of publication of application: **22.05.2013**
(21) Application number: 11806911.1
(22) Date of filing: 15.07.2011
(51) Int. Cl.: C12N 15/09, B42D 15/10, C12Q 1/68, G06K 19/06

(54) **HOLDER THAT HOLDS IDENTIFYING INFORMATION FOR IDENTIFYING AN IDENTIFICATION SUBJECT, AND USE THEREFOR**

(30) Priority: 16.07.2010 JP 2010162335
(71) Applicant: NGK Insulators, Ltd., Nagoya-shi, Aichi 467-8530 (JP)
(72) Inventor: HIROTA Toshikazu, Nagoya-shi Aichi 467-8530 (JP); NIWA Kousuke, Nagoya-shi Aichi 467-8530 (JP)
(74) Representative: Naylor, Matthew John
(86) International application number: PCT/JP2011/066246
(87) International publication number: WO 2012/008581

(57) **Abstract**

Disclosed are a carrier that carries identifying information having favorable identification performance and a use thereof. The carrier carries identifying information for identifying an identification subject, and comprises one or more pieces of DNA having a thymine-rich base sequence and having, as the identifying information, an identifying base sequence pre-associated with the identification subject.

SEQUENCE LISTING

<110>  NGK INSULATORS, LTD.

<120>  Carrier having Identification Information of Subject

<130>  K10-262-PCT

<160>  115

<170>  PatentIn version 3.3

<210>  1
<211>  23
<212>  DNA
<213>  Artificial

<220>
<223>  probe

<400>  1
tgttctctga ccaatgaatc tgc                                              23


<210>  2
<211>  23
<212>  DNA
<213>  Artificial

<220>
<223>  probe

<400>  2
tggaactggg aacgctttag atg                                              23


<210>  3
<211>  23
<212>  DNA
<213>  Artificial

<220>
<223>  probe

<400>  3
ttcgcttcgt tgtaatttcg gac                                              23


<210>  4
<211>  23
<212>  DNA
<213>  Artificial

<220>
<223>  probe

<400>  4
aggcatccta agaaatcgct act                                              23


<210>  5
<211>  23
<212>  DNA
<213>  Artificial

<220>
<223>  probe

<400>  5
tagcccagtg atttatgaca tgc                                              23


<210>  6
<211>  23
<212>  DNA
<213>  Artificial

<220>
<223>  probe

<400>  6
cgctctggtt actattggac gtt                                              23


<210>  7
<211>  23
<212>  DNA
<213>  Artificial

<220>
<223>  probe

<400>  7
tagccaactc taaataacgg acg                                              23


<210>  8
<211>  23
<212>  DNA
<213>  Artificial

<220>
<223>  probe

<400>  8
ttcggttgtc gatatgagga tct                                              23


<210>  9
<211>  23
<212>  DNA
<213>  Artificial

<220>
<223>  probe

<400>  9
gggggtact tcatacaaga tgc                                               23


<210>  10
<211>  23
<212>  DNA
<213>  Artificial

<220>
<223>  probe

<400>  10
gagtagcagg caaataccct aga                                              23

<210>  11
<211>  23
<212>  DNA
<213>  Artificial

<220>
<223>  probe

<400>  11
gcctattaag gtctacgtca tcg                                              23


<210>  12
<211>  23
<212>  DNA
<213>  Artificial

<220>
<223>  probe

<400>  12
agtcatacag tgaggaccaa atg                                              23


<210>  13
<211>  23
<212>  DNA
<213>  Artificial

<220>
<223>  probe

<400>  13
cattcgacat aagctgttga tgc                                              23


<210>  14
<211>  23
<212>  DNA
<213>  Artificial

<220>
<223>  probe

<400>  14
tgctcactta cattacgtcc atg                                              23


<210>  15
<211>  23
<212>  DNA
<213>  Artificial

<220>
<223>  probe

<400>  15
tacacctatc aactcgtaga gca                                              23


<210>  16
<211>  23
<212>  DNA

<213>  Artificial

<220>
<223>  probe

<400>  16
aggtccggta gtaatttagg tgc                                               23


<210>  17
<211>  23
<212>  DNA
<213>  Artificial

<220>
<223>  probe

<400>  17
tgcactctga tatatacagg cca                                               23


<210>  18
<211>  23
<212>  DNA
<213>  Artificial

<220>
<223>  probe

<400>  18
gcagccctta tagataacgg gac                                               23


<210>  19
<211>  23
<212>  DNA
<213>  Artificial

<220>
<223>  probe

<400>  19
gaagccatga tactgttcag ggt                                               23


<210>  20
<211>  23
<212>  DNA
<213>  Artificial

<220>
<223>  probe

<400>  20
tattctacca acgacatcac tgc                                               23


<210>  21
<211>  23
<212>  DNA
<213>  Artificial

<220>
<223>  probe

<400>  21
ccatcagtta ttcggaggga ctc                                              23


<210>  22
<211>  23
<212>  DNA
<213>  Artificial

<220>
<223>  probe

<400>  22
ccatatccga ttattagcga cgg                                              23


<210>  23
<211>  23
<212>  DNA
<213>  Artificial

<220>
<223>  probe

<400>  23
catctccaag aattgaccca cca                                              23


<210>  24
<211>  23
<212>  DNA
<213>  Artificial

<220>
<223>  probe

<400>  24
ccgtcgtgtt attaaagacc cct                                              23


<210>  25
<211>  23
<212>  DNA
<213>  Artificial

<220>
<223>  probe

<400>  25
gaaggatcgc ttttatctgg cat                                              23


<210>  26
<211>  23
<212>  DNA
<213>  Artificial

<220>
<223>  probe

<400>  26
catttgtcag gtacagtcca ctt                                              23


<210>  27

<211>  23
<212>  DNA
<213>  Artificial

<220>
<223>  probe

<400>  27
gcccacactc ttacttatcg act                                              23


<210>  28
<211>  23
<212>  DNA
<213>  Artificial

<220>
<223>  probe

<400>  28
cgctgttact gtaagcgtac tag                                              23


<210>  29
<211>  23
<212>  DNA
<213>  Artificial

<220>
<223>  probe

<400>  29
cgcgattcct attgattgat ccc                                              23


<210>  30
<211>  23
<212>  DNA
<213>  Artificial

<220>
<223>  probe

<400>  30
ccgtctgggt taaagattgc tag                                              23


<210>  31
<211>  23
<212>  DNA
<213>  Artificial

<220>
<223>  probe

<400>  31
agtcagtcca aatctcagga tgg                                              23


<210>  32
<211>  23
<212>  DNA
<213>  Artificial

<220>

<223>  probe

<400>  32
cgcctaaatg aaactcactc tgc                                                23


<210>  33
<211>  23
<212>  DNA
<213>  Artificial

<220>
<223>  probe

<400>  33
ggggtcaaac caacaattga tct                                                23


<210>  34
<211>  23
<212>  DNA
<213>  Artificial

<220>
<223>  probe

<400>  34
gcccattgat agaattacga ggc                                                23


<210>  35
<211>  23
<212>  DNA
<213>  Artificial

<220>
<223>  probe

<400>  35
atgccgttgt caagagttat ggt                                                23


<210>  36
<211>  23
<212>  DNA
<213>  Artificial

<220>
<223>  probe

<400>  36
tgccggctat cgtaagtata tgc                                                23


<210>  37
<211>  23
<212>  DNA
<213>  Artificial

<220>
<223>  probe

<400>  37
gcacctcata ccttcataga gca                                                23

<210>  38
<211>  23
<212>  DNA
<213>  Artificial

<220>
<223>  probe

<400>  38
cgcgacattt agtccaggag atg                                              23


<210>  39
<211>  23
<212>  DNA
<213>  Artificial

<220>
<223>  probe

<400>  39
ctagtccatt gtaacgaagg cca                                              23


<210>  40
<211>  23
<212>  DNA
<213>  Artificial

<220>
<223>  probe

<400>  40
agacaattag aatcagtgcc cct                                              23


<210>  41
<211>  23
<212>  DNA
<213>  Artificial

<220>
<223>  probe

<400>  41
gcattgaggt attgttgctc cca                                              23


<210>  42
<211>  23
<212>  DNA
<213>  Artificial

<220>
<223>  probe

<400>  42
cgagagtctg taatagccga tgc                                              23


<210>  43
<211>  23
<212>  DNA
<213>  Artificial

<220>
<223>  probe

<400>  43
tgccgtgata cttaactacg cta                                              23


<210>  44
<211>  23
<212>  DNA
<213>  Artificial

<220>
<223>  probe

<400>  44
gagtccgcaa aaatatagga ggc                                              23


<210>  45
<211>  23
<212>  DNA
<213>  Artificial

<220>
<223>  probe

<400>  45
gcctcacata actggagaaa cct                                              23


<210>  46
<211>  23
<212>  DNA
<213>  Artificial

<220>
<223>  probe

<400>  46
cgccaatgac aataagttga ggc                                              23


<210>  47
<211>  23
<212>  DNA
<213>  Artificial

<220>
<223>  probe

<400>  47
cgcgatataa cattaaccga ggc                                              23


<210>  48
<211>  23
<212>  DNA
<213>  Artificial

<220>
<223>  probe

<400>  48

cacgcttagt tcctacctta ggc                                              23

<210>  49
<211>  23
<212>  DNA
<213>  Artificial

<220>
<223>  probe

<400>  49
cgcgtcgaat tacttaatca cca                                              23

<210>  50
<211>  23
<212>  DNA
<213>  Artificial

<220>
<223>  probe

<400>  50
gggataggta ttatgctcca gcc                                              23

<210>  51
<211>  23
<212>  DNA
<213>  Artificial

<220>
<223>  probe

<400>  51
cgccattata caacggttca tgc                                              23

<210>  52
<211>  23
<212>  DNA
<213>  Artificial

<220>
<223>  probe

<400>  52
gcctatatga accaagccac tgc                                              23

<210>  53
<211>  23
<212>  DNA
<213>  Artificial

<220>
<223>  probe

<400>  53
cgccgtcagt acttgtatag atg                                              23

<210>  54
<211>  23

<212>  DNA
<213>  Artificial

<220>
<223>  probe

<400>  54
gtcggtatcg aaaaggtact gca                                              23


<210>  55
<211>  23
<212>  DNA
<213>  Artificial

<220>
<223>  probe

<400>  55
aggcagttca acctatatct gcg                                              23


<210>  56
<211>  23
<212>  DNA
<213>  Artificial

<220>
<223>  probe

<400>  56
ggtcgtaaca ttgagaggag acg                                              23


<210>  57
<211>  23
<212>  DNA
<213>  Artificial

<220>
<223>  probe

<400>  57
ggcgatttat tgctaactgg cta                                              23


<210>  58
<211>  23
<212>  DNA
<213>  Artificial

<220>
<223>  probe

<400>  58
gcactaccgc taactatacg cta                                              23


<210>  59
<211>  23
<212>  DNA
<213>  Artificial

<220>
<223>  probe

<400>  59
ggctcgtagt actccttaca tgc                                               23


<210>  60
<211>  23
<212>  DNA
<213>  Artificial

<220>
<223>  probe

<400>  60
ggctctacaa acttgtgtcc atg                                               23


<210>  61
<211>  23
<212>  DNA
<213>  Artificial

<220>
<223>  probe

<400>  61
ggtggagtga atctcactag act                                               23


<210>  62
<211>  23
<212>  DNA
<213>  Artificial

<220>
<223>  probe

<400>  62
ctagcacaat taatcaatcc gcc                                               23


<210>  63
<211>  23
<212>  DNA
<213>  Artificial

<220>
<223>  probe

<400>  63
gcagctgaat tgctatgatc acc                                               23


<210>  64
<211>  23
<212>  DNA
<213>  Artificial

<220>
<223>  probe

<400>  64
gcctatagtg tcgattgtcc tcg                                               23

<210>  65
<211>  23
<212>  DNA
<213>  Artificial

<220>
<223>  probe

<400>  65
cgatcacgga ttaatgtcac ccc                                              23


<210>  66
<211>  23
<212>  DNA
<213>  Artificial

<220>
<223>  probe

<400>  66
aagagattta acttgagctc gcc                                              23


<210>  67
<211>  23
<212>  DNA
<213>  Artificial

<220>
<223>  probe

<400>  67
tttgttgttc gatatcaggc gtg                                              23


<210>  68
<211>  23
<212>  DNA
<213>  Artificial

<220>
<223>  probe

<400>  68
gcccgggaat agattataac gca                                              23


<210>  69
<211>  23
<212>  DNA
<213>  Artificial

<220>
<223>  probe

<400>  69
gcatttttag taatccgagc gcc                                              23


<210>  70
<211>  23
<212>  DNA
<213>  Artificial

<220>
<223>  probe

<400>  70
catggataag ttttcaagct gcg                                              23


<210>  71
<211>  23
<212>  DNA
<213>  Artificial

<220>
<223>  probe

<400>  71
gagacaggta aaccctcaga gca                                              23


<210>  72
<211>  23
<212>  DNA
<213>  Artificial

<220>
<223>  probe

<400>  72
tagcacccgt taaaacggaa atg                                              23


<210>  73
<211>  23
<212>  DNA
<213>  Artificial

<220>
<223>  probe

<400>  73
tatgtttagt tgttgaaccg gcg                                              23


<210>  74
<211>  23
<212>  DNA
<213>  Artificial

<220>
<223>  probe

<400>  74
cgatcagctc tatttccctc cca                                              23


<210>  75
<211>  23
<212>  DNA
<213>  Artificial

<220>
<223>  probe

<400>  75
agtcagttaa tcagacgtga gca                                              23

<210>  76
<211>  23
<212>  DNA
<213>  Artificial

<220>
<223>  probe

<400>  76
tggcaataca ataacgtatc gcg                                              23


<210>  77
<211>  23
<212>  DNA
<213>  Artificial

<220>
<223>  probe

<400>  77
cgcagtttgc aagaacgaac aaa                                              23


<210>  78
<211>  23
<212>  DNA
<213>  Artificial

<220>
<223>  probe

<400>  78
cgcgataatt gatacctacg ggc                                              23


<210>  79
<211>  23
<212>  DNA
<213>  Artificial

<220>
<223>  probe

<400>  79
ggggtgtgag agctttttag acg                                              23


<210>  80
<211>  23
<212>  DNA
<213>  Artificial

<220>
<223>  probe

<400>  80
gggatccgta acaagtgtgt tag                                              23


<210>  81
<211>  23
<212>  DNA

<213>  Artificial

<220>
<223>  probe

<400>  81
accactatga ttgaggaaac gcg                                              23


<210>  82
<211>  23
<212>  DNA
<213>  Artificial

<220>
<223>  probe

<400>  82
cgtctttagt atcaaccctc cgc                                              23


<210>  83
<211>  23
<212>  DNA
<213>  Artificial

<220>
<223>  probe

<400>  83
gcatacgaac ttctatatcg gcg                                              23


<210>  84
<211>  23
<212>  DNA
<213>  Artificial

<220>
<223>  probe

<400>  84
ccgtgtgtat gagtatgaca gca                                              23


<210>  85
<211>  23
<212>  DNA
<213>  Artificial

<220>
<223>  probe

<400>  85
tgctgtcttc gtgttttacc tag                                              23


<210>  86
<211>  23
<212>  DNA
<213>  Artificial

<220>
<223>  probe

<400>  86
cgatcatgta aagctaactc gcg                                              23


<210>  87
<211>  23
<212>  DNA
<213>  Artificial

<220>
<223>  probe

<400>  87
tgccgtcatt taaacgtaag ggt                                              23


<210>  88
<211>  23
<212>  DNA
<213>  Artificial

<220>
<223>  probe

<400>  88
tggcaattac agttgttaac gca                                              23


<210>  89
<211>  23
<212>  DNA
<213>  Artificial

<220>
<223>  probe

<400>  89
gagtcgaaga cctcctccta ctc                                              23


<210>  90
<211>  23
<212>  DNA
<213>  Artificial

<220>
<223>  probe

<400>  90
atgccaatat gtactcgtga ctc                                              23


<210>  91
<211>  23
<212>  DNA
<213>  Artificial

<220>
<223>  probe

<400>  91
gcatatagtg acggtaaggc gaa                                              23


<210>  92

<211>  23
<212>  DNA
<213>  Artificial

<220>
<223>  probe

<400>  92
gcctcacttg taataagcgg gac                                              23


<210>  93
<211>  23
<212>  DNA
<213>  Artificial

<220>
<223>  probe

<400>  93
gtcccaaaag cttcttacgg acg                                              23


<210>  94
<211>  23
<212>  DNA
<213>  Artificial

<220>
<223>  probe

<400>  94
ctaggtacaa caccaactgt ctc                                              23


<210>  95
<211>  23
<212>  DNA
<213>  Artificial

<220>
<223>  probe

<400>  95
tgccggttat acctttaagg acg                                              23


<210>  96
<211>  23
<212>  DNA
<213>  Artificial

<220>
<223>  probe

<400>  96
ggctggttaa atgtaaatcc gcg                                              23


<210>  97
<211>  23
<212>  DNA
<213>  Artificial

<220>

<223>  probe

<400>  97
cgcggtacta ttagaaaggg cta                                               23


<210>  98
<211>  23
<212>  DNA
<213>  Artificial

<220>
<223>  probe

<400>  98
agtcgcttaa ttactccgga tgg                                               23


<210>  99
<211>  23
<212>  DNA
<213>  Artificial

<220>
<223>  probe

<400>  99
cgctgttggt attaccttcc tcg                                               23


<210>  100
<211>  23
<212>  DNA
<213>  Artificial

<220>
<223>  probe

<400>  100
tgcagtgtaa gcaactattg tct                                               23


<210>  101
<211>  23
<212>  DNA
<213>  Artificial

<220>
<223>  Sequence for Identification

<400>  101
ttcgcttcgt tgtaatttcg gac                                               23


<210>  102
<211>  23
<212>  DNA
<213>  Artificial

<220>
<223>  Sequence for Identification

<400>  102
gagacaggta aaccctcaga gca                                               23

<210>  103
<211>  23
<212>  DNA
<213>  Artificial

<220>
<223>  Sequence for Identification

<400>  103
gagacaggaa aacccacaga gca                                              23


<210>  104
<211>  33
<212>  DNA
<213>  Artificial

<220>
<223>  Sequence for Identification

<400>  104
tttttttttt ttcgcttcgt tgtaatttcg gac                                   33


<210>  105
<211>  33
<212>  DNA
<213>  Artificial

<220>
<223>  Sequence for Identification

<400>  105
tttttttttt gagacaggta aaccctcaga gca                                   33


<210>  106
<211>  33
<212>  DNA
<213>  Artificial

<220>
<223>  Sequence for Identification

<400>  106
tttttttttt gagacaggaa aacccacaga gca                                   33


<210>  107
<211>  33
<212>  DNA
<213>  Artificial

<220>
<223>  Probe

<400>  107
ttcgcttcgt tgtaatttcg gacttttttt ttt                                   33


<210>  108
<211>  33
<212>  DNA
<213>  Artificial

<220>
<223>  Probe

<400>  108
gagacaggta aaccctcaga gcattttttt ttt                                    33


<210>  109
<211>  33
<212>  DNA
<213>  Artificial

<220>
<223>  Probe

<400>  109
gagacaggaa aacccacaga gcattttttt ttt                                    33


<210>  110
<211>  43
<212>  DNA
<213>  Artificial

<220>
<223>  Probe

<400>  110
tttttttttt ttcgcttcgt tgtaatttcg gacttttttt ttt                         43


<210>  111
<211>  43
<212>  DNA
<213>  Artificial

<220>
<223>  Probe

<400>  111
tttttttttt gagacaggta aaccctcaga gcattttttt ttt                         43


<210>  112
<211>  43
<212>  DNA
<213>  Artificial

<220>
<223>  Probe

<400>  112
tttttttttt gagacaggaa aacccacaga gcattttttt ttt                         43


<210>  113
<211>  23
<212>  DNA
<213>  Artificial

<220>
<223>  Probe

<400>  113

gtccgaaatt acaacgaagc gaa                                              23

<210>  114
<211>  23
<212>  DNA
<213>  Artificial

<220>
<223>  Probe

<400>  114
tgctctgagg gtttacctgt ctc                                              23

<210>  115
<211>  23
<212>  DNA
<213>  Artificial

<220>
<223>  Probe

<400>  115
tgctctgtgg gttttcctgt ctc                                              23

## Description

### TECHNICAL FIELD

This application claims priority on Japanese Patent Application No. 2010-162335 filed on July 16, 2010, the entire contents of which are incorporated herein.
The present application relates to a carrier that carries identifying information for identifying an identification subject and to the use thereof, and more particularly, relates to a carrier that carries identifying information containing DNA and to the use thereof.

### BACKGROUND ART

In recent years, various types of distributed goods, such as finished products, components (including intermediate products), marine products, agricultural products, bank notes and securities, are being required to be controlled from the viewpoints of quality, safety, tampering prevention, determination of authenticity and the like.

Monitoring and control of the distribution and transport of these various types of identification subjects is normally carried out by imparting fixed or pre-associated identifying information to the identification subject, and identifying that identifying information at appropriate times. For example, a technology has been proposed for avoiding forgery and tampering and the like by fabricating a DNA ink using a DNA base sequence, printing the DNA ink onto an identification subject, and detecting the DNA base sequence at appropriate times (Japanese Patent Application Laid-open No. 2008-187991).

### SUMMARY OF INVENTION

Identification subjects retaining identifying information are exposed to various environments during the course of distribution and transport. On the other hand, in the identifying of an identification subject using DNA, it is necessary to maintain a high level of identification performance. In order to accomplish this, the DNA is required to be stably held in the identification subject without undergoing deterioration.

DNA may be damaged by ultraviolet light of a wavelength of about 200 nm to 300 nm. In addition, there is also the possibility of DNA being separated from an identification subject or being severed on the identification subject when subjected to frictional external force or changes in temperature or humidity and the like during distribution.

In such cases, it may become difficult to accurately detect the imparted DNA, thereby resulting in the risk of a decrease in identification performance.

In addition, it is important that detection be able to be carried out easily and rapidly in order to identify an identification subject with good precision.

Therefore, the disclosure of the present description provides a carrier that carries identifying information having favorable identification performance and the use thereof.

The inventors of the present invention conducted extensive studies in order to hold DNA used as identifying information for identifying an identification subject on a carrier thereof in a chemically stable manner while inhibiting separation thereof As a result, it was found that immobilization performance of the DNA to the carrier can be improved and the DNA can be immobilized in a chemically stable manner while inhibiting separation attributable to friction and the like by using thymine, which is a type of base that composes DNA, or a derivative thereof. The following aspects are provided according to the disclosure of the present description.

(1) A carrier that carries identifying information for identifying an identification subject, and comprises one or more pieces of DNA having: an identifying base sequence pre-associated with the identification subject; and a thymine-rich base sequence.
(2) The carrier described in (1), wherein the thymine-rich base sequence is provided on one or both of a 5'-end and/or 3'-end of the DNA.
(3) The carrier described in (1) or (2), wherein a T (thymine) content of the identifying base sequence (number of thymine bases in the identifying base sequence/total number of bases of the identifying base sequence × 100) is less than 50%.
(4) The carrier described in (3), wherein the T content of the identifying base sequence is 10% or less.
(5) The carrier described in any of (1) to (4), wherein the thymine-rich base sequence is composed of two or more consecutive thymine bases.
(6) The carrier described in any of (1) to (5), wherein the thymine-rich base sequence is composed of 3 or 4 or more consecutive thymine bases.
(7) The carrier described in any of (1) to (5), wherein the thymine-rich base sequence is composed of 5 or 6 or more consecutive thymine bases.
(8) The carrier described in any of (1) to (7), wherein the identifying base sequence is selected from SEQ ID NO: 1 to SEQ ID NO: 100 and complementary sequences thereof.
(9) The carrier described in any of (1) to (8), wherein the one or more pieces of DNA are bound in the carrier by chemical bonding.
(10) The carrier described in (9), wherein a plurality of dots containing the one or more pieces of DNA form a visually identifiable pattern.
(11) The carrier described in (10), wherein two or more types of the patterns are provided.
(12) The carrier described in (11), wherein the two or more types of the patterns are formed of dots composed of one or more pieces of DNA having the identifying base sequence that is not shared between the patterns.
(13) The carrier described in (12), wherein at least portions of the two or more types of patterns are arranged overlapping each other.
(14) The carrier described in any of (1) to (13), further comprising a cover that covers a portion, which is provided with the DNA, of the carrier.
(15) A method for identifying an identification subject, comprising steps of:
   bringing one or more probes having a base sequence complementary to the identifying base sequence into contact with the DNA of the carrier described in any of (1) to (14) imparted to the identification subject; and
   detecting a hybridization product of the identifying base sequence and the probe.
(16) An identification kit of an identification subject, comprising:
   the carrier described in any of (1) to (14); and
   one or more probes having a base sequence complementary to the identifying base sequence.

### DESCRIPTION OF EMBODIMENTS

The disclosure of the present description relates to a carrier for identifying an identification subject that uses an identifying base sequence in DNA as identifying information, and to the use thereof. According to the carrier disclosed in the present description, since decomposition of DNA that carries identifying information is inhibited, and DNA is held on the carrier while inhibiting separation and the like thereof, a carrier having favorable identification performance is provided that has superior identification accuracy, precision and reproducibility. In addition, as a result of DNA that retains identifying information being held on the carrier in this manner, the DNA can be detected both rapidly and easily. In addition, since an identifying base sequence is used that is selected from base sequences represented by SEQ ID NO: 1 to SEQ ID NO: 100 and complementary sequences thereof, highly specific identification performance is ensured and an identification subject can be identified rapidly with high selectivity. The following provides a detailed explanation of embodiments of the disclosure of the present description.

### (Carrier)

The carrier disclosed in the present description is a carrier that carries identifying information for identifying an identification subject, and can be provided with one or more pieces of DNA having an identifying base sequence that is pre-associated with the identification subject as the above-mentioned identifying information, and a thymine-rich base sequence in at least a portion thereof.

### (Identification Subject)

There are no particular limitations on the identification subject identified by the carrier disclosed in the present description (to be referred to as the present carrier), and examples thereof include various types of distributed goods and media. Distributed goods refer to all goods distributed commercially or non-commercially. Examples include various types of industrial products, components (including intermediate products), marine products, agricultural products, works of art and books, as well as bank notes and securities. In addition, other examples include ID cards for personal identification as well as various types of certificates.

The present carrier can adopt various forms. For example, the present carrier may be that which holds identifying information on various shapes of supports such as films, sheets or substrates. In this case, the present carrier is immobilized on the identification subject by chemical means such as adhesion or by other physical means. In addition, the present carrier may be a part of the identification subject. For example, identifying information may be held on a portion of an identification subject.

Examples of the form of the above-mentioned support include films, flat plates, particles, molded articles (such as beads, strips, wells or strips of multi-well plates, tubes, mesh, continuously blown foam, membranes, paper, needles, fibers, plates, slides or cell culturing vessels) and latex. In addition, there are no particular restrictions on the size thereof. When considering detection, the region where the identifying information is imparted is preferably flat.

There are no particular restrictions on the material that composes the support on which identifying information in the form of DNA is held or the site of the identification subject (such as a support) provided it enables DNA to be immobilized thereon by physical adsorption or chemical bonding, and is able to withstand normal hybridization conditions. Specific examples include materials that are insoluble in solvents used in nucleic acid immobilization and hybridization and are in the state of a solid or gel at room temperature or within a temperature range in the vicinity thereof (for example, 0°C to 100°C).

Examples of such materials of a support and the like include plastics, inorganic polymers, metals, natural polymers and ceramics. There are no particular restrictions on the above-mentioned plastics provided they are able to immobilize biomolecules by irradiating with ultraviolet light, and specific examples thereof include thermoplastic resins, thermosetting resins and copolymers. More specifically, examples of thermoplastic resins include ionomers (styrene-based and olefin-based), polynorbomene, polyacetal, polyarylate, polyether ether ketone, polyethylene oxide, polyoxymethylene, polyethylene terephthalate, polycarbonate, polystyrene, polysulfone, polyparamethylstyrene, poly(allylamine), polyphenylene ether, polyphenylene sulfide, polybutadiene, polybutylene terephthalate, polypropylene, polymethylpentene, polyether sulfone, polyphenylene sulfide, polyoxybenzoyl, polyoxyethylene, cellulose acetate, polydimethylsiloxane, polyisobutylene, cellulose triacetate, poly-p-phenylene terephthalamide, polyisoprene, polyacrylonitrile, polymethylpentene, chlorine-based plastics (polyvinyl chloride, polyethylene chloride, polypropylene chloride, polyvinylidene chloride), fluorine-based plastics (tetrafluoroethylene, polychlorotrifluoroethylene, polyvinylidene fluoride), polyamides (Nylon 6, Nylon 66), polyamidoamide, polyimides (thermoplastic polyimide, polyetherimide), polyethylene-based plastics (chlorinated, high density, low density), polyvinyl-based plastics (polyvinyl chloride, polyvinyl acetate, polyparavinylphenol, polyvinyl alcohol, polyvinyl ether, polyvinyl butyral, polyvinyl formal), liquid crystal polymers (polyester-based liquid crystal polymers), acrylate-based plastics (aminopolyacrylamide, polymethyl acrylate, polymethyl methacrylate, polyethyl methacrylate, polybutyl methacrylate), and thermoplastic elastomers (styrene-based, olefin-based, urethane-based, polyester-based, polyamide-based, 1,2-polybutadiene-based, vinyl chloride-based, fluorine-based, polyionomer-based, chlorinated polyethylene-based, silicone-based).

In addition, examples of thermosetting resins include epoxy, polyxylene, polyguanamine, polydiallylphthalate, polyvinyl ester, polyphenol, unsaturated polyester, polyfuran, polyimide, polyurethane, polymaleic acid, melamine, urea, alkyd, benzoguanamine, polycyanate and polyisocyanate resins.

Moreover, examples of copolymers include isobutylene-maleic anhydride copolymers, acrylonitrile-styrene acrylate copolymers, acrylonitrile-EPDM styrene copolymers, acrylonitrile-styrene copolymers, acrylonitrile-butadiene-styrene copolymers, butadiene-styrene-methyl methacrylate copolymers, ethylene-vinyl chloride copolymers, ethylene-vinyl acetate copolymers, ethylene-ethyl acrylate copolymers, acrylonitrile-butadiene-styrene copolymers, poly(ether ether ketone) copolymers, ethylene fluoride-polypropylene copolymers, tetrafluoroethylene-perfluoroalkylvinyl ether copolymers, and tetrafluoroethylene-ethylene copolymers.

Particularly preferable examples of the above-mentioned synthetic resins include polycarbonate, polymethyl methacrylate, acrylonitrile-butadiene-styrene copolymers, polyethylene, polyethylene terephthalate, polyphenol, polystyrene, polyacrylonitrile, polyvinyl chloride and aramid.

In addition, a synthetic resin can be used in which a dye, colorant, plasticizer, pigment, polymerization inhibitor, surface modifier, stabilizer, adhesion promoter, hardener, dispersant or ultraviolet shielding agent and the like is added as necessary to the above-mentioned synthetic resins. Moreover, the above-mentioned synthetic resins may be obtained by laminating different types of the above-mentioned synthetic resins in order to maintain shape, or may be composed of a single synthetic resin. In addition, a polymer alloy may be used that is obtained by mixing two or more types of the above-mentioned synthetic resins. Moreover, fibers such as leaf vein fibers, fruit fibers, animal hair fibers, silk fibers, down fibers, chitin, chitosan or asbestos may be mixed into the above-mentioned synthetic resins.

Specific examples of inorganic polymers include glass, quartz, carbon, silica gel and graphite. Specific examples of metals include gold, platinum, silver, copper, iron, aluminum, magnets and permamagnets. Examples of natural polymers include polyamino acids, cellulose, chitin, chitosan, alginic acid and derivatives thereof. Specific examples of ceramics include apatite, alumina, silica, silicon carbide, silicon nitride and boron carbide.

Although DNA may be immobilized directly on the above-mentioned support, an immobilization phase may be further imparted to the support and the like for the purpose of immobilization. This immobilization phase may be loaded by simply using physical adhesion or may be chemically loaded through covalent bonds, provided the immobilization phase is loaded on the above-mentioned support. In addition, the above-mentioned immobilization phase may also be loaded onto the entire surface of the support or may be loaded onto a portion thereof as necessary. Examples of the immobilization phase include low molecular weight organic compounds in addition to the materials previously explained as materials of the support and the like. Specific examples of the above-mentioned low molecular weight organic compounds include carbodiimide group-containing compounds, isocyanate group-containing compounds, nitrogen yperite group-containing compounds, aldehyde group-containing compounds and amino group-containing compounds.

The immobilization phase is preferably loaded as a film on the support and the like. A known method can be used to load the immobilization phase on the support and the like as a film, examples of which include spraying, immersion, brushing, stamping, vapor deposition and coating using a film coater.

For example, in a method for introducing carbodiimide groups (resin) onto the entire surface of a glass support and the like, the support and the like is first immersed in a solution obtained by dissolving an amino-substituted organoalkoxysilane such as 3-aminopropyltriethoxysilane in a suitable solvent for roughly 2 hours to 3 hours under temperature conditions of about 70°C to 80°C, and is then taken out of the solution, rinsed with water and heat dried for about 4 hours to 5 hours at about 100°C to 120°C. After drying, the support is immersed in a suitable solvent and carbodiimide resin is added followed by stirring for about 12 hours under temperature conditions of about 30°C to 170°C and washing. In addition, functional groups other than the nucleic acid bonding groups of the amino groups of the above-mentioned 3-aminopropyltriethoxysilane and the nitrogen yperite groups can be allowed to react using a suitable solvent to introduce the nitrogen yperite groups onto the surface of the glass support and the like.

In addition, in the case of groups other than amino groups on the glass support and the like or in the case the support and the like is composed of a material other than glass, the introduction of various functional groups on the surface of each of the materials listed as examples in the above-mentioned explanation of the support is typically carried out according to the prior art, and since methods thereof are known, functional groups can be introduced into the surface of the support and the like using such known methods.

Moreover, some of the examples of plastic supports and the like listed above already have functional groups like those previously described on the surface thereof, and in such cases, can be used as is in the production of a support and the like without having to introduce functional groups on the surface thereof. In addition, these plastic supports can also be used to produce the above-mentioned support and the like by further introducing functional groups.

In addition, a known photopolymerization initiator can also be mixed into the material of the above-mentioned support and the like or immobilization phase. Mixing in a photopolymerization inhibitor makes it possible to improve reactivity during immobilization of nucleic acid by irradiating with electromagnetic waves such as ultraviolet light.

### (Identifying Information)

In the present description, identifying information refers to information for identifying an identification subject. Identifying information is contained in one or more pieces of DNA. Moreover, identifying information may be contained in a pattern composed by imparting this DNA onto a carrier. This pattern will be subsequently described.

In the present description, DNA refers to a polymer of deoxyribonucleotides respectively having adenine (A), thymine (T), cytosine (C) and guanine (G) as bases thereof.

Identifying information is associated with a single identification subject, and identifying information is composed of one or more identifying base sequences provided in one or more pieces of DNA. Thus, a single identification subject may be identified by a single piece of DNA, or may be identified by two or more pieces of DNA.

A single piece of DNA has a single identifying base sequence. The identifying base sequence is pre-associated with a single identification subject. The identification subject is identified by the identifying information as a result of this association. The identifying base sequence may or may not be unique to an identification subject.

The case of being unique to an identification subject refers to, for example, the case in which, when an identification subject has a characteristic base sequence or mutation in its genome, the characteristic base sequence per se is used for the identifying base sequence.

The identifying base sequence may be of natural origin or may be artificially designed. In the case of using an artificially designed base sequence as an identifying base sequence, detection of an identification subject can be carried out rapidly and with high accuracy by hybridization by using an artificial base sequence that composes a collection of bases such that hybridization and detection can be carried out reliably under common hybridization conditions without the preliminary occurrence of mutual mishybridization.

Examples of such artificial base sequences include the base sequences represented by SEQ ID NO: 1 to SEQ ID NO: 100 and complementary base sequences thereof These base sequences are all of the same length, have a melting temperature (Tm) of 40°C to 80°C and preferably 50°C to 70°C, and allow the obtaining of uniform hybridization results during hybridization under the same conditions. When using two or more types of artificial base sequences simultaneously, the melting temperatures thereof are preferably as close together as possible.

Furthermore, although a melting temperature calculated according to the GC% method, Wallace method or method in compliance with Current Protocols in Molecular Biology (described in Shujunsha Publishing Co., Ltd., Biological Experiments Illustrated 3, Increasingly Prevalent PCR, p. 25) can be employed for the melting temperature, it is preferably calculated according to the Nearest Neighbor method since this method is capable of taking into consideration the range of melting temperatures in the present invention as well as the effects of base sequence concentration. Melting temperatures according to the Nearest Neighbor method can be easily acquired with software used in conjunction with Visual OMP (Tomy Digital Biology Co., Ltd.) or software (OligoCalculator;http://www.ngrl.co.jp/tool/ngrl_tool.html) provided by Nihon Gene Research Laboratories, Inc. (http://www.ngrl.co.jp/).

The identifying sequence in such artificial base sequences is also referred to as a normally orthogonalized sequence, and is designed by, for example, calculating the continuous matching length with respect to a DNA sequence of a prescribed base length obtained from random numbers, prediction of melting temperature according to the Nearest Neighbor method, hamming distance or prediction of secondary structure. Normal orthogonalized sequences refer to base sequences of nucleic acids that are designed so as to have a uniform melting temperature, or in other words, have melting temperatures that fall within a constant range, do not inhibit the formation of hybrids with complementary sequences as a result of the nucleic acids per se forming intramolecular structures, and do not form stable hybrids except for those formed with base sequences complementary thereto. Sequences contained in a single normal orthogonalized sequence group are unlikely to undergo or do not undergo reactions with sequences other than a desired combination thereof or reactions within its own sequence. In addition, normal orthogonalized sequences have the property of enabling nucleic acids to be amplified quantitatively in an amount corresponding to the initial amount of nucleic acid having the normal orthogonalized sequence when amplified by PCR without being affected by problems such as cross-hybridization as previously described. Normal orthogonalized sequences as mentioned above are described in detail in H. Yoshida and A. Suyama, "Solution to 3-SAT by Breadth First Research", DIMACS VI. 54, 9-20 (2000) and in Japanese Patent Application No. 2003-108126. Normal orthogonalized sequences can be designed by using the methods described in these documents.

Identifying information may be composed by one or more identifying base sequences. Identification of even higher accuracy can be made possible by composing identifying information with two or more pieces of DNA having respectively different identification base sequences. In addition, even if a fixed number of artificial base sequences is used, a number of identification subjects that is greater than that fixed number can be identified by combining those artificial base sequences.

The identifying base sequence preferably has a lower ratio of thymine bases than the thymine-rich base sequence to be subsequently described. Namely, the T (thymine) content of the identifying base sequence (as determined by the number of thymine bases in the identifying base sequence/the total number of bases of the identifying base sequence × 100) is preferably less than 50%. This is because, if thymine is present in the identifying base sequence, in addition to an increase in the immobilized amount and/or the amount that reacts with a probe, there is increased susceptibility to deterioration caused by irradiation with ultraviolet light and the like following immobilization. Although there are no particular limitations on the thymine ratio provided a desired immobilized amount and/or reacted amount and inhibition of deterioration are obtained, it is preferably 40% or less, more preferably 30% or less, even more preferably 20% or less, further preferably 10% or less, more further preferably 5% or less, even more further preferably 1% or less, and most preferably 0%.

DNA has a thymine-rich base sequence together with a single identifying base sequence. As will be subsequently described, thymine is thought to be involved in chemical bonding of DNA to the carrier, and as a result of having a thymine-rich base sequence, DNA can be easily and firmly bound to a support.

A thymine-rich base sequence refers to a base sequence in which the thymine (T) content thereof is higher than other regions of DNA (such as an identifying base sequence). More specifically, this refers to a contiguous base sequence containing on both ends thereof thymine bases specified by two thymine (T) bases selected in a DNA base sequence having a higher thymine content than other regions. More preferably, the thymine (T) content (as determined by the number of thymine bases in the above-mentioned contiguous base sequence/the total number of bases in the contiguous base sequence × 100) is 50% or more. The thymine (T) content as described above is more preferably 60% or more, even more preferably 70% or more, further preferably 80% or more, more further preferably 90% or more, even more further preferably 95% or more, still even more preferably 98% or more, and most preferably 100%. The thymine-rich base sequence preferably contains thymine bases at intervals without any consecutive thymine bases. In addition, the thymine-rich base sequence preferably contains a base sequence composed of two or more consecutive thymine bases. More preferably, the thymine-rich base sequence is composed of a base sequence of consecutive thymine bases. A base sequence containing consecutive thymine bases preferably has 3 or 4 or more consecutive thymine bases, more preferably 5 or 6 or more consecutive thymine bases, even more preferably 7 or 8 or more consecutive thymine bases, and still more preferably 9 or 10 or more consecutive thymine bases. Furthermore, the thymine-rich base sequence may extend throughout or nearly throughout the DNA.

Although the thymine-rich base sequence may be at any site in the DNA, it is preferably not a portion of the identifying base sequence. The thymine-rich base sequence is preferably provided separate from the identifying base sequence. In addition, although the thymine-rich base sequence may be provided containing or not containing the 5'-end and/or 3'-end of the DNA, it may also be provided on the 5'-end and/or 3'-end. In addition, it may also be provided in a central portion of the DNA. It is preferably provided on the 5'-end and/or 3'-end.

As a result of the DNA being provided with a thymine-rich base sequence, the DNA is bound to the present carrier by chemical bonding.

As a result of one or more pieces of DNA being retained on a carrier support, the identifying base sequence in the DNA functions as identifying information. The identifying information is preferably retained on the support and the like accompanied by a suitable pattern (two-dimensional shape). Namely, although there are no particular limitations thereon, the pattern may be formed of symbols, letters, numbers, bar codes, images or a combination thereof. The pattern is also preferably able to be visualized.

DNA on the carrier may be retained in the form of one or a plurality of dots on the carrier. Individual dots are composed of one or more pieces of DNA. In the case of using a single piece of DNA to identify a single identification subject, a single dot is composed entirely of that single piece of DNA. In addition, in the case of using two or more dots to identify a single identification subject, a single dot may be composed of a single piece of DNA or be composed of two or more pieces of DNA. Alternatively, two or more dots respectively composed of DNA may be combined.

A prescribed visible pattern may also be composed by gathering together a plurality of dots. Composing the pattern with dots not only makes it possible to reduce the amount of DNA required for detection, but since DNA concentration within the dots can be increased, detection sensitivity can be enhanced and highly accurate detection can be carried out easily and rapidly. A single pattern is normally composed in order to specify a single identification subject. That single pattern is formed of dots composed of one or more pieces of DNA. Furthermore, a single pattern or two or more patterns may be provided for a single identification subject.

Two or more patterns may also be simultaneously provided on a single support or identification subject. In this case, in order to hold the two or more patterns in a compact manner, the two or more patterns are preferably arranged so that at least portions thereof overlap each other. The patterns may be arranged so that individual dots belonging to each pattern do not overlap at the location where the patterns overlap, or they may be arranged so that individual dots belonging to each pattern overlap. In addition, the location where the patterns overlap may be composed of only the dots belonging to either of the patterns. Moreover, none of the dots belonging to either pattern may be arranged at the location where the patterns overlap. This is because the patterns per se can still be confirmed even in this state.

Furthermore, two or more patterns may be provided for identifying a single identification subject, or may be provided to respectively identify separate identification subjects. In either case, the patterns are preferably formed of dots composed of one or more pieces of DNA having the above-mentioned identifying base sequence that is not shared between the patterns.

Furthermore, technology such as a known DNA microarray can be applied for the technology for forming dots containing DNA on a support and the like. Namely, a liquid obtained by dispersing or dissolving DNA may be spotted on a support using a known technique such as an inkjet method or pin method.

The present carrier can include a cover that covers the portion provided with DNA. The providing of such a cover makes it possible to inhibit separation of DNA caused by friction and inhibit decomposition by irradiation with ultraviolet light. The cover may be adhered to the surface of the above-mentioned carrier that is imparted with identifying information. Namely, the cover may be in the form of a film or sheet that is adhered by having a pressure-sensitive adhesive layer. In addition, the cover may cover the surface of the above-mentioned carrier that is imparted with identifying information while separated by a prescribed distance there from. Moreover, the cover may be a cover that is separated by a prescribed distance from the surface of the above-mentioned carrier imparted with identifying information and forms a cavity on the above-mentioned surface. Moreover, in the case of a cover that forms such a cavity, the cavity can also be used as a cavity for hybridization. In this case, the cavity can be provided with an opening that allows injection of a probe solution and the like for carrying out hybridization. Alternatively, the opening can be provided so as to be able to be formed at appropriate times. For example, a weakened portion may be provided so that the opening is formed by a pressing force from the outside.

Moreover, a region where the identifying information is retained is preferably formed lower than the surrounding area in order to protect DNA serving as identifying information on the present carrier from physical stimulation. For example, in the case the support is provided with a recess having a bottom surface, the identifying information is preferably retained in the recess. In addition, the recess can also be used as a cavity for carrying out hybridization.

Providing a cavity for carrying out hybridization in the present carrier makes it possible to carry out a contact step easily when identifying an identification subject as will be subsequently described.

In the case the present carrier is provided with identifying information on a support that is to be separately immobilized on an identification subject, the support is preferably provided with means for being immobilized on the identification subject. An example of immobilization means is an adhesive layer or pressure-sensitive adhesive layer. This type of immobilization means is effective in the case of the present carrier being in the form of a film or sheet. In addition, in the case the present carrier is in the form of a tag, immobilization means such as locking or engagement can be provided in addition to an adhesive layer or pressure-sensitive adhesive layer. The immobilization means preferably allows the present carrier to be separated from the identification subject. Enabling the present carrier to be separated from the identification subject allows identification of the identification subject to be subsequently described to be carried out easily.

### (Identification Method)

The following provides an explanation of a method for identifying an identification subject using the present carrier. Namely, the identification method of an identification subject disclosed in the present description can be provided with a step of bringing one or more probes having a base sequence complementary to the above-mentioned identifying base sequence into contact with the above-mentioned identifying information on the present carrier imparted to the identification subject, and a step of detecting a hybridization product of the identifying base sequence and the probe. According to the present identification method, the identification subject can be identified with favorable accuracy since favorable identification performance is ensured for the present carrier. The identification step can also be directly applied to methods for controlling, monitoring, authenticating, identifying or tracking the identification subject.

### (Contact Step)

The contact step can be a step of bringing one or more probes having a base sequence complementary to the identifying base sequence contained in the identifying information into contact with this identifying information on the present carrier imparted to the identification subject. In the contact step, a hybridization product can be formed with the probe when an identifying base sequence is provided that is pre-associated with the identification subject. Namely, the present contact step is carried out for the purpose of carrying out hybridization between the identifying base sequence contained in DNA and the probe. The probe may only provide a probe corresponding to the identifying base sequence preliminarily imparted to the identification subject, or may provide a probe composed to be universally applicable to a large number of identification subjects.

Although the probe may only be complementary to a degree that allows the formation of a hybridization product with the identifying base sequence, it is preferably completely complementary. When the identifying base sequence is selected from base sequences represented by SEQ ID NO: 1 to SEQ ID NO: 100 and complementary base sequences thereof, the probe is selected from base sequences represented by SEQ ID NO: 1 to SEQ ID NO: 100 and complementary base sequences thereof.

The probe is preferably labeled for the purpose of subsequent detection. A conventionally known label can be suitably selected and used for the label. The label may be formed of various types of dyes such as fluorescent materials that emit a fluorescence signal when the label per se is excited, or may be formed of substances that emit various types of signals by combining with a second component by an enzymatic reaction or antigen-antibody reaction. Typically, a fluorescently-labeled substance such as Cy3, Alexa 555, Cy5 or Alexa 647 can be used. In addition, detection may be also be employed that is based on the generation of color by treating with a substrate and the like by combining biotin and streptoavidin HPR.

Although the probe may include a probe unique to the identification subject, it may also be formed of a probe set composed to be universally applicable to a large number of identification subjects. Since a probe having a base sequence represented by SEQ ID NO: 1 to SEQ ID NO: 100 and base sequences complementary thereto is free of the occurrence of mutual mishybridization, if the present probe uses such a base sequence for the identifying sequence thereof, a probe set can be composed that can be universally applied.

There are no particular limitations on the conditions of the contact step. An ordinary hybridization medium can be used. The temperature can be set to a suitable temperature. For example, in the case of using a highly selective identifying base sequence such as a base sequence represented by SEQ ID NO: 1 to SEQ ID NO: 100 or a base sequence complementary thereto, a temperature of 30°C to 80°C, and preferably 30°C to 40°C, can be used in the case of using a base sequence represented by SEQ ID NO: 1 to SEQ ID NO: 100 or a base sequence complementary thereto. The temperature is even more preferably 35°C to 40°C. In addition, the duration of the contact step is preferably made to be 1 second to 1 hour, more preferably 1 second to 5 minutes and even more preferably 1 second to 1 minute. The contact step can be carried out at a temperature of preferably 30°C to 40°C and more preferably 35°C to 40°C for a duration of preferably 1 second to 5 minutes and more preferably 1 second to 1 minute.

Furthermore, when carrying out the contact step, the present carrier may be separated from the identification subject. In the case the present carrier is immobilized on the identification subject with separable immobilization means, the contact step can be carried out with the present carrier separated from the identification subject. In addition, the contact step may also be carried out on the identification subject when possible. An example of this is the case in which a cavity for carrying out hybridization is provided in the present carrier.

Excess probe is preferably removed by washing prior to the subsequent detection step. Since DNA serving as identifying information is immobilized on a support and the like, even if excess probe is washed off, the hybridization product is retained on the support and the like.

### (Detection Step)

The detection step can be a step of detection of a hybridization product between the identifying base sequence in the above-mentioned identifying information and the above-mentioned probe. The identification subject can be identified by detecting this hybridization product. There are no particular limitations on the method used to detect the hybridization product in the detection step. In the case a linking molecule has a label, that label may be detected. In addition, double strands may be detected using an electrical detection method and the like.

The identification subject is identified when the hybridization product is detected. Namely, since the identification subject is judged to be identical, the identification subject can be determined to be free of tampering, free of substitution or free of damage and the like. In addition, when a hybridization product is not detected, the identification subject is either determined to not be present or not be identical. Namely, the identification subject is determined to have been lost, tampered with or damaged and the like.

In the case one or more pieces of DNA form a visible pattern for the identifying information, the identification subject can be easily identified by visually confirming that pattern, thereby identifying the identification subject. In addition, in the case of using a base sequence represented by SEQ ID NO: 1 to SEQ ID NO: 100 or a complementary sequence thereof, since the hybridization product is formed with high selectivity, identification can be carried out rapidly and highly accurately. Moreover, highly accurate identification is also possible in the case two or more pieces of DNA, or in other words, two or more identifying base sequences, are associated with a single identification subject.

Although there are no particular limitations thereon, the amount of time required for the detection step can be 1 second to 1 hour. In the present method, since hybridization and detection are possible at 40°C or lower (e.g. about 37°C) in comparison with an ordinary detection temperature (50°C to 70°C) by using, for example, a highly selective identifying base sequence selected from the base sequences represented by SEQ ID NO: 1 to SEQ ID NO: 100 and complementary sequences thereof, the speed of the detection step can be accelerated. The duration of the detection step is more preferably 1 second to 5 minutes and even more preferably 1 second to 1 minute.

As has been explained above, according to the present identification method, a contact step and a detection step can be carried out easily and rapidly for the present carrier having favorable identification performance. Thus, highly accurate identification is possible. In addition, this identification method demonstrates similar effects even when applied to control methods, monitoring methods or authentication methods and the like relating to the distribution and storage of various types of goods.

### (Identification Kit)

The identification kit of an identification subject disclosed in the present description can be provided with the present carrier and one or more probes having a base sequence complementary to an identifying base sequence. According to this kit, identification can be carried out using the present carrier. Various previously described aspects of the present carrier and probe in the present identification kit can be applied directly to the identification kit.

### (Production Method of Present Carrier)

The method used to produce the present carrier can be provided with a step of supplying DNA having an identifying base sequence and a thymine-rich base sequence to the surface of the above-mentioned support and the like, and immobilizing the DNA and support while bringing the two into contact with each other. More specifically, in the immobilization step, for example, the DNA is normally supplied in a form contained in water or a buffer so that the activity of the immobilized DNA is maintained during the contact reaction of the two components. In addition, DNA can be immobilized by irradiating the two components with electromagnetic waves either during the time they are in contact or after they have made contact. In addition, a known photopolymerization initiator can be mixed into the above-mentioned water or buffer.

Ultraviolet light having a wavelength of 220 nm to 380 nm is preferable for the electromagnetic waves used for immobilization. Irradiation with ultraviolet light including a wavelength of 280 nm is particularly preferable. More specifically, the ultraviolet light may be ultraviolet light having a broad waveform that includes a wavelength of 280 nm. The dose is preferably 10 mJ/cm² to 5000 mJ/cm² and more preferably 100 mJ/cm² to 2000 mJ/cm². Preferably, the dose is 200 mJ/cm² or more.

In addition, the above-mentioned nucleic acid solution can be dried after spotting prior to irradiation with ultraviolet light. The above-mentioned nucleic acid solution may be dried by air-drying or drying by heating. In the case of drying by heating, the temperature is normally 30°C to 100°C and preferably 35°C to 45°C.

DNA may be chemically or physically bound to a known compound such as a carbodiimide resin, nitrogen ypteride, polyamino acid or nitrocellulose, and a mixture thereof may be contacted with a support in this state and immobilized, and immobilization at this time may be carried out after having irradiated with the above-mentioned electromagnetic waves.

In the present description, although examples of methods used to supply a small amount of DNA, and normally water or buffer containing DNA, to a support and the like in the form of dots include a method using a dispenser, a method using a pin and a method using an inkjet, the present invention is not limited thereto. In addition, such devices for supplying a small amount of a solution are generally available commercially, and these devices can also be used in the present invention.

Moreover, a blocking step may also be provided. Blocking can be carried out on the present carrier by bringing a support and the like into contact with an excess amount of bovine serum albumin (BSA), casein or salmon sperm DNA and the like as necessary.

As has been explained above, according to the present production method, a DNA carrier can be obtained that inhibits deterioration and separation of DNA and exhibits favorable identification performance.

### Examples

Although the following provides an explanation of examples that embody the present invention, the disclosure of the present description is not limited to the following examples.

### Example 1

In the present example, oligonucleotides were synthesized that were composed of three types of sequences composed of an identifying base sequence of a 23 mer artificially designed oligonucleotide having a low ratio of thymine (T) bases, that having a high ratio of thymine bases and that completely free of thymine bases, and a sequence in which polyT (thymine-rich base sequence) was connected to the 5'-end and/or 3'-end of these sequences. These oligonucleotides were spotted onto a resin substrate and immobilized thereon. In order to evaluate the effects of additional ultraviolet radiation after immobilizing the spots on the identification performance (detection performance) of an identification subject, a hybridization reaction was carried out with a probe before and after irradiating with ultraviolet light, followed by a comparison of the intensities of the fluorescence signals. Furthermore, the additional irradiation with ultraviolet light was carried out by continuously irradiating a support on which the oligonucleotides were immobilized with ultraviolet light of about 200 nm to 300 nm for an extended period of time.

### (1) Fabrication of Immobilized Oligonucleotides

Aqueous solutions obtained by dissolving preliminarily prepared synthetic oligonucleotides (DNA) (Nihon Gene Research. Laboratories, Inc.: see Table 1) were spotted onto a substrate made of a thermoplastic resin in the form of polycarbonate (25 mm ± 0.05 mm × 76 mm ± 0.05 mm) using the Geneshot Spotter manufactured by NGK Insulators, Ltd. The oligonucleotides were formed of three types composed of Oligo 1-1, Oligo 2-1 and Oligo 3-1 having 10, 2 and 0 thymine groups in the identifying base sequence (SEQ ID NO: 101 to SEQ ID NO: 103), and three types composed of Oligo 1-2, Oligo 2-2 and Oligo 3-2 having PolyT composed of 10 thymine bases respectively coupled to the 5'-ends of the above oligonucleotides (SEQ ID NO: 104 to SEQ ID NO: 106). In addition, the oligonucleotides were also formed of three types composed of Oligo 1-3, Oligo 2-3 and Oligo 3-3 having PolyT composed of 10 thymine bases respectively coupled to the 3'-ends of the oligonucleotides composed of base sequences represented by SEQ ID NO: 101 to SEQ ID NO: 103 (SEQ ID NO: 107 to SEQ ID NO: 109). Moreover, the oligonucleotides were also formed of three types composed of Oligo 1-4, Oligo 2-4 and Oligo 3-4 having PolyT composed of 10 thymine bases respectively coupled to both the 5'-end and the 3'-end of the oligonucleotides composed of base sequences represented by SEQ ID NO: 101 to SEQ ID NO: 103 (SEQ ID NO: 110 to SEQ ID NO: 112).

**[Table 1]**

| Type of Oligonucleotide | Base Sequence 5'→3' | No. of Internal T | No. of Terminal T |
|---|---|---|---|
| Oligo 1-1 | TTCGCTTCGTTGTAATTTCGGAC | 10 | 0 |
| Oligo 2-1 | GAGACAGGTAAACCCTCAGAGCA | 2 | 0 |
| Oligo 3-1 | GAGACAGGAAAACCCACAGAGCA | 0 | 0 |
| Oligo 1-2 | TTTTTTTTTTTTCGCTTCGTTGTAATTTCGGAC | 10 | 10 |
| Oligo 2-2 | TTTTTTTTTTGAGACAGGTAAACCCTCAGAGCA | 2 | 10 |
| Oligo 3-2 | TTTTTTTTTTGAGACAGGAAAACCCACAGAGCA | 0 | 10 |
| Oligo 1-3 | TTCGCTTCGTTGTAATTTCGGACTTTTTTTTTT | 10 | 10 |
| Oligo 2-3 | GAGACAGGTAAACCCTCAGAGCATTTTTTTTTT | 2 | 10 |
| Oligo 3-3 | GAGACAGGAAAACCCACAGAGCA TTTTTTTTTT | 0 | 10 |
| Oligo 1-4 | TTTTTTTTTTTTCGCTTCGTTGTAATTTCGGACTTTTTTTTTT | 10 | 10 + 10 |
| Oligo 2-4 | TTTTTTTTTTGAGACAGGTAAACCCTCAGAGCATTTTTTTTTT | 2 | 10 + 10 |
| Oligo 3-4 | TTTTTTTTTTGAGACAGGAAAACCCACAGAGCATTTTTTTTTT | 0 | 10 + 10 |

Furthermore, aqueous solutions of the synthetic oligonucleotides were prepared as indicated below.
(a) Spotting aqueous solutions using additive 1 : SSC-based
   Equivolume mixtures of 6xSSC (obtained by diluting 20xSSC manufactured by Invitrogen Corp.) and each of the oligonucleotides at a concentration of 100 pmol/µl (respective final concentrations: 50 pmol/µl, 3xSSC)
(b) Spotting aqueous solutions using additive 2: PBS-based
   Equivolume mixtures of 2xPBS (obtained by diluting 10×PBS manufactured by Invitrogen Corp.) and each of the oligonucleotides at a concentration of 100 µmol/µl (respective final concentrations: 50 pmol/µl, 1×PBS)

After spotting, the synthetic oligonucleotides were immobilized on the support (substrate) according to the following procedure. Namely, the spotted substrates were placed in a UV irradiation device (Spectroline Laboratories, Ltd., XL-1500UV Crosslinker) and irradiated with ultraviolet light at 600 mJ/cm². Next, after placing the substrates in a slide rack and vertically shaking 50 times in a 3% to 5% aqueous solution of BSA, the substrates were vertically shaken 10 times in sterile water followed by centrifuging (1000 rpm × 2 min) to remove liquid.

### (2) Reaction with Probe DNA

Synthetic DNA, in which the 5'-end thereof had been labeled with Cy3 (Nihon Gene Research Laboratories, Inc., refer to the three types of sequences indicated below: SEQ ID NO: 113 to SEQ ID NO: 115), were used as probes for detecting the oligonucleotides on the supports retaining the synthetic DNA fabricated in (1).

**[Table 2]**

| Probe | Base Sequence 5'→3' |
|---|---|
| For detecting Oligo 1 | GTCCGAAATTACAACGAAGCGAA |
| For detecting Oligo 2 | TGCTCTGAGGGTTTACCTGTCTC |
| For detecting Oligo 3 | TGCTCTGTGGGTTTTCCTGTCTC |

To begin with, reaction solutions using the above-mentioned three types of probes were prepared with the compositions indicated below and reacted with the supports (substrates) retaining the synthetic oligonucleotides. Details of the procedure are indicated below.

### (Preparation of Probe DNA Solutions)

| | |
|---|---|
| Probe mixture (2.5 nM each)* | 1.5 µl |
| Hybri solution (×2)* | 9.0 µl |
| Milli-Q water | 7.5 µl |
| Total | 18.0 µl |

| | |
|---|---|
| * Probe mixture composition (2.5 nM each) | |

| | |
|---|---|
| Probe 1 (100 nM) | 10 µl |
| Probe 2 (100 nM) | 10 µl |
| Probe 3 (100 nM) | 10 µl |
| TE (pH 8.0) | 370 ul |
| Total | 400 µl |

| | |
|---|---|
| * Hybri solution composition (x2) | |

| | |
|---|---|
| 20xSSC | 2.0 ml |
| 10% SDS | 0.8 ml |
| 100% formamide | 12.0 ml |
| 100 mM EDTA | 0.8 ml |
| Milli-Q | 24.4 ml |
| Total | 40.0 ml |

### (Reaction of Substrates and Probe DNA Solutions)

After heating the prepared reaction probe DNA solutions for 1 minute at 90°C using the GeneAmp PCR System 9700 manufactured by Applied Biosystems Inc., the solutions were heated for 1 minute at 80°C using a heat block (Taitec Corp., DTU-N). 9 µl aliquots of the above-mentioned probe solutions were then applied to a spotting area on the substrates and allowed to react by allowing to stand undisturbed for 60 minutes at 37°C using Thermo Block slides for the Eppendorf Thermomixer® Comfort and Eppendorf Thermostat™ Plus (Eppendorf AG) in order to prevent drying.

### (Preparation of Post-Reaction Substrate Washing Solution)

| | |
|---|---|
| Milli-Q | 188.0 ml |
| 20xSSC | 10.0 ml |
| 10% SDS | 2.0 ml |
| Total | 200.0 ml |

### (Washing of Excess Probe on Substrates)

The above-mentioned washing solution was transferred to a glass staining vat. The substrates following completion of reaction with the probe DNA solutions were immersed in the vat and vertically shaken for 5 minutes. The substrates were then transferred to a glass staining vat containing sterile water and shaken vertically for 1 minute. Next, the substrates were dried by centrifuging for 1 minute at 2000 rpm to remove any moisture remaining thereon.

### (3) Fabrication of Substrates Obtained by Additionally Irradiating Supports

Fabricated in (1) with Ultraviolet Light and Reaction Using Probe DNA of Substrates
In order to confirm the long-term storage stability of the array, the substrates fabricated in (1) were placed in a UV irradiation device (Spectroline Laboratories, Ltd., XL-1500UV Crosslinker) to fabricate substrates additionally irradiated with ultraviolet light (irradiated with 600 mJ/cm² and 1200 mJ/cm², respectively).

Probe DNA was then reacted with each of the substrates obtained by additionally irradiating with ultraviolet light using the same method as that used in (2), followed by washing off excess probe remaining on the substrates.

### (4) Data Analysis

### (Detection of Fluorescence Using a Scanner)

Fluorescent images of the substrate surfaces were acquired for each of the reacted substrates obtained in (2) and (3) by suitably adjusting the exposure time using Array WoRx manufactured by Applied Precision Inc. Moreover, fluorescence signals of the resulting images were digitized using GenePix Pro. Results were then compared for fluorescence intensity of each of the spots on the substrates obtained in (2). Those results are shown in the table below.

**[Table 3]**

| Oligonucleotide | Spot Fluorescence Intensity | | No. of Internal T | No. of Terminal T |
|---|---|---|---|---|
| | SSC-based | PBS-based | | |
| Oligo 1-1 | 153 | 89 | 10 | 0 |
| Oligo 2-1 | 265 | 122 | 2 | 0 |
| Oligo 3-1 | 2284 | 287 | 0 | 0 |
| Oligo 1-2 | 408 | 269 | 10 | 10 |
| Oligo 2-2 | 1942 | 1039 | 2 | 10 |
| Oligo 3-2 | 25773 | 4518 | 0 | 10 |
| Oligo 1-3 | 501 | 235 | 10 | 10 |
| Oligo 2-3 | 2312 | 978 | 2 | 10 |
| Oligo 3-3 | 24597 | 4331 | 0 | 10 |
| Oligo 1-4 | 388 | 199 | 10 | 10 + 10 |
| Oligo 2-4 | 1844 | 775 | 2 | 10 + 10 |
| Oligo 3-4 | 20018 | 3976 | 0 | 10 + 10 |

As shown in Table 3, results were confirmed such that, irrespective of the method used to prepare the oligonucleotide aqueous solutions (SSC-based or PBS-based), as the number of thymine bases in the identifying base sequence of the oligonucleotides decreased (in the order of Oligo 1-1, 2-1, 3-1, Oligo 1-2, 2-2, 3-2, Oligo 1-3, 2-3, 3-3 and Oligo 1-4, 2-4, 3-4), the amount of probe that reacted increased. In addition, when the effects of the presence or absence of polyT bound to an end or ends of the oligonucleotides on the amount of probe that reacted were confirmed, results were confirmed such that the amount of probe that reacted in the case of those oligonucleotides to which polyT was bound (Oligo 1-2 to Oligo 1-4, Oligo 2-2 to Oligo 2-4 and Oligo 3-2 to Oligo 3-4) ranged from several times to about 10 times more than those oligonucleotides to which polyT was not bound (Oligo 1-1, Oligo 2-1 and Oligo 3-1).

In addition, results were compared for the fluorescence intensities of each of the spots on the substrates obtained according to (3) above (SSC-based and PBS-based). The results are shown in the table below while incorporating the results of (2) above.

**[Table 4]**

| Oligonucleotide | Additional UV Irradiation/SSC-based | | | No. of internal T | No. of terminal T |
|---|---|---|---|---|---|
| | None | 600 mJ/cm² | 1200 mJ/cm² | | |
| Oligo 1-1 | 153 | 12 | 1 | 10 | 0 |
| Oligo 2-1 | 265 | 44 | 38 | 2 | 0 |
| Oligo 3-1 | 2284 | 232 | 88 | 0 | 0 |
| Oligo 1-2 | 408 | 25 | 2 | 10 | 10 |
| Oligo 2-2 | 1942 | 148 | 37 | 2 | 10 |
| Oligo 3-2 | 25773 | 13799 | 8109 | 0 | 10 |

**[Table 5]**

| Oligonucleotide | Additional UV Irradiation/PBS-based | | | No. of internal T | No. of terminal T |
|---|---|---|---|---|---|
| | None | 600 mJ/cm² | 1200 mJ/cm² | | |
| Oligo 1-1 | 89 | 10 | 3 | 10 | 0 |
| Oligo 2-1 | 122 | 21 | 4 | 2 | 0 |
| Oligo 3-1 | 287 | 134 | 19 | 0 | 0 |
| Oligo 1-2 | 269 | 52 | 6 | 10 | 10 |
| Oligo 2-2 | 1039 | 103 | 11 | 2 | 10 |
| Oligo 3-2 | 4518 | 1859 | 190 | 0 | 10 |

As shown in Table 4, in the case of employing an SSC-based method for the preparation method of the oligonucleotide aqueous solutions, the ratio of the decrease in fluorescence intensity of the spots additionally irradiated with ultraviolet light in Oligo 3-2 (number of thymine bases in identifying base sequence: 0, polyT present) was confirmed to be able to be inhibited to roughly several tenths to about one-third that of the prior art.

On the basis of the above results, in sequences having only a small number of thymine bases in the identifying base sequence of an oligonucleotide and in which polyT is bound to an end thereof, the effects of ultraviolet light on the oligonucleotide immobilized on a substrate can be reduced, and long-term storage of oligonucleotides and arrays on which oligonucleotides have been immobilized was determined to be favorable.

In addition, as is shown in Table 5, in the case of employing a PBS-based method for the preparation method of the oligonucleotide aqueous solutions, the ratio of the decrease in fluorescence intensity of the spots additionally irradiated with ultraviolet light was somewhat larger in comparison with the SSC-based method, and preparation of spotting aqueous solutions using the SSC-based method was determined to be desirable.

### SEQUENCE LISTINGS FREE TEXT

SEQ ID NO: 1 to SEQ ID NO: 115: Probes

## Claims

1. A carrier that carries identifying information for identifying an identification subject, and comprises one or more pieces of DNA having:
an identifying base sequence pre-associated with the identification subject; and
a thymine-rich base sequence.

2. The carrier according to claim 1, wherein the thymine-rich base sequence is provided on one or both of a 5'-end and/or 3'-end of the DNA.

3. The carrier according to claim 1 or 2, wherein a T (thymine) content of the identifying base sequence (number of thymine bases in the identifying base sequence/total number of bases of the identifying base sequence × 100) is less than 50%.

4. The carrier according to claim 3, wherein the T content of the identifying base sequence is 10% or less.

5. The carrier according to any of claims 1 to 4, wherein the thymine-rich base sequence is composed of two or more consecutive thymine bases.

6. The carrier according to any of claims 1 to 5, wherein the thymine-rich base sequence is composed of 3 or 4 or more consecutive thymine bases.

7. The carrier according to any of claims 1 to 6, wherein the thymine-rich base sequence is composed of 5 or 6 or more consecutive thymine bases.

8. The carrier according to any of claims 1 to 7, wherein the identifying base sequence is selected from SEQ ID NO: 1 to SEQ ID NO: 100 and complementary sequences thereof

9. The carrier according to any of claims 1 to 8, wherein the one or more pieces of DNA are bound to the carrier by chemical bonding.

10. The carrier according to claim 9, wherein a plurality of dots containing the one or more pieces of DNA forms a visually identifiable pattern.

11. The carrier according to claim 10, wherein two or more types of the patterns are provided.

12. The carrier according to claim 11, wherein the two or more types of the patterns are formed of dots composed of one or more pieces of DNA having the identifying base sequence that is not shared between the patterns.

13. The carrier according to claim 12, wherein at least portions of the two or more types of patterns are arranged overlapping each other.

14. The carrier according to any of claims 1 to 13, further comprising a cover that covers a portion, which is provided with the DNA, of the carrier.

15. A method for identifying an identification subject, comprising steps of:
bringing one or more probes having a base sequence complementary to the identifying base sequence into contact with the DNA of the carrier according to any of claims 1 to 14 imparted to the identification subject; and
detecting a hybridization product of the identifying base sequence and the probe.

16. An identification kit of an identification subject, comprising:
the carrier according to any of claims 1 to 14; and
one or more probes having a base sequence complementary to the identifying base sequence.
